# DEMANDE DE BREVET EUROPEEN

(11) **EP 0 740 936 A2**
(43) Date de publication de la demande: **06.11.1996**
(21) Numéro de dépôt: 96400931.0
(22) Date de dépôt: 02.05.1996
(51) Int. Cl.: A61K 31/35

(54) **Utilisation des dérivés du benzopyrane pour l' obtention de compositions pharmaceutiques destinées au traitement des pathologies liées a l'énchangeur Cl-/HCO3-,Na+ indépendant**

(30) Priorité: 05.05.1995 FR 9505361
(71) Demandeur: ADIR ET COMPAGNIE, F-92415 Courbevoie Cédex (FR)
(72) Inventeur: Durant, Ludovic, 49300 Cholet (FR); Babingui, Jean-Paul, 44000 Nantes (FR); Moulin, Claudie, 44000 Nantes (FR); Robert-Piessard, Sylvie, 44200 Nantes (FR); Le Baut, Guillaume, 44230 Saint Sébastien sur Loire (FR); Scalbert, Elisabeth, 92100 Boulogne (FR); Caignard, Daniel-Henri, 75015 Paris (FR); Renard, Pierre, 78000 Versailles (FR)

(57) **Abrégé**

L'invention concerne l'utilisation des composés de formule (I) : 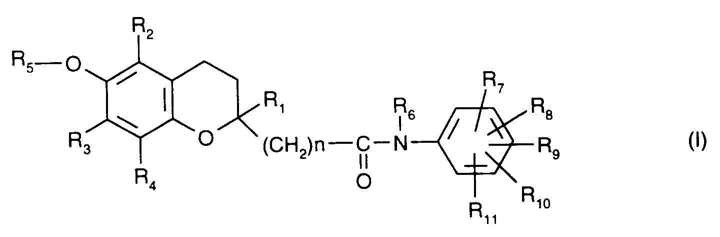 dans laquelle R₁, R₂, R₃, R₄, R₅, R₆, R₇, R₈, R₉, R₁₀, R₁₁ et n sont tels que définis dans la description.

pour l'obtention de compositions pharmaceutiques destinées à la prévention et au traitement des troubles liés à l'échangeur Cl⁻/HCO₃⁻, Na⁺ indépendant.

## Description

L'invention concerne l'utilisation des dérivés du benzopyrane pour l'obtention de compositions pharmaceutiques destinées au traitement des pathologies liées à l'échangeur Cl⁻/HCO₃⁻, Na⁺ indépendant.

L'échangeur Cl⁻/HCO₃⁻, Na⁺ indépendant ("anion exchanger", ci-après désigné "AE") catalyse une sortie d'ion bicarbonate HCO₃⁻ de la cellule contre une entrée de chlore Cl⁻ dans la cellule. Il est électriquement neutre .

Cet échangeur est notamment connu pour être impliqué dans la régulation du pH intracellulaire, du volume cellulaire et de la concentration intracellulaire de chlore (Cl⁻). Cependant, au niveau de l'érythrocyte il joue également un rôle très spécifique en permettant le transport du CO₂ des cellules aux poumons sous la forme de HCO₃-plasmatique.

Concernant la régulation du pH intracellulaire, cet échangeur constitue le seul processus physiologique d'extrusion hors de la cellule d'équivalents basiques médié par une protéine dont la structure moléculaire est connue. Il est généralement activé par l'alcalose intracellulaire.

Le prototype de l'échange Cl⁻/HCO₃⁻, Na⁺ indépendant, dénommé "AE1" a été mis en évidence initialement au niveau de la membrane érythrocytaire, mais il a servi de base à l'identification de deux autres gènes codant pour des "AE2" et "AE3". Les AE1, AE2 et AE3 présentent une homologie de 80 % au niveau de leur partie membranaire -COOH terminale (partie essentielle pour le fonctionnement de l'échangeur) mais présentent également une spécificité d'expression tissulaire très importante (Alper S.L. Cell. Physiol. Biochem, 1994, 4 : 265-281).

L'inhibition de l'échangeur AE au niveau du cardiomyocyte est bénéfique lors d'une ischémie myocardique pour prévenir l'acidose intracellulaire (par rétention intracellulaire des HCO₃⁻) et ses conséquences ioniques (Na⁺ et Ca⁺⁺) et métaboliques. En effet, même si théoriquement, l'AE (type AE3) du cardiomyocyte se doit d'être activé essentiellement par l'alcalose intracellulaire, il a été montré récemment que cet AE est activé par la stimulation purinergique ou β₁-adrenergique, engendrant alors une acidification intracellulaire (Pucéat M. et al., J. Physiol., 1991, 444 : 241-256 ; Désilets M. et al., Circ Res, 1994, 75 : 862-869). Lors d'une ischémie myocardique les concentrations des médiateurs sont généralement accrues (ex : catécholamines, ATP et angiotensine II) et entraînent dans ce cas, une hyperactivation de l'AE cardiomyocytaire qui contribue à l'acidification néfaste à l'intégrité cellulaire en complément de l'acidification métabolique déjà présente lors de l'ischémie.

L'invention concerne l'utilisation des composés de formule (I) : 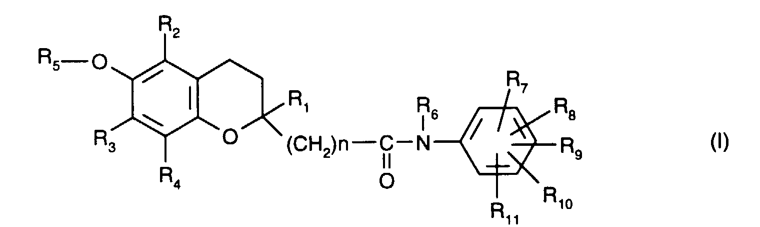
dans laquelle
n représente 0 ou 1,
R₁, R₂, R₃ et R₄, identiques ou différents, représentent, chacun indépendamment l'un de l'autre, un hydrogène ou un alkyl ;
R₅ représente un hydrogène ou un radical choisi parmi alkyl, alkylcarbonyl, alkoxyalkyl, alkoxycarbonyl, alkoxycarbonylalkyl et carboxyalkyl ;
R₆ représente un hydrogène ou un radical choisi parmi alkyl, phényl et phénylalkyl ;
R₇, R₈ et R₉ représentent, chacun indépendamment l'un de l'autre, un radical choisi parmi halogène, alkyl, alkyl substitué par un ou plusieurs halogènes, alkoxy, hydroxy, alkoxycarbonyl et carboxy ;
et R₁₀ et R₁₁ représentent, chacun indépendamment l'un de l'autre, un hydrogène ou un radical choisi parmi halogène, alkyl, alkyl substitué par un ou plusieurs halogènes, alkoxy, hydroxy, alkoxycarbonyl et carboxy ;
leurs énantiomères et diastéréoisomères et leurs sels d'addition à une base pharmaceutiquement acceptable,
étant entendu que
les termes "alkyl" et "alkoxy" désignent des groupements linéaires ou ramifiés de 1 à 8 atomes de carbone,
pour l'obtention de compositions pharmaceutiques destinées à la prévention et au traitement des troubles liés à l'échangeur Cl⁻/HCO₃⁻, Na⁺ indépendant.

Plus particulièrement, l'invention concerne l'utilisation pour l'obtention de compositions pharmaceutiques destinées à la prévention et au traitement des pathologies liées à l'échangeur Cl⁻/HCO₃⁻, Na⁺ indépendant, des composés de formule (I) dans lesquels pris ensemble ou séparément le cas échéant :
n représente 0,
n représente 1,
R₁, R₂, R₃, R₄ représentent un alkyl,
R₅ représente un hydrogène,
R₅ représente un acétyl,
R₆ représente un hydrogène,
et R₁₀ et R₁₁ représentent un hydrogène,
leurs énantiomères et diastéréoisomères et leurs sels d'addition à un base pharmaceutiquement acceptable.

De façon particulière, les radicaux alkyls présents dans la formule (I) peuvent être choisis parmi méthyl, éthyl, n-propyl, isopropyl, n-butyl, isobutyl, sec-butyl, tertio-butyl, pentyl, hexyl, heptyl et octyl, par exemple méthyl.

Les radicaux alkoxy présents dans la formule (I) peuvent être choisis parmi méthoxy, éthoxy, n-propoxy, isopropoxy, n-butoxy, iso-butoxy, sec-butoxy, tertio-butoxy, pentyloxy, hexyloxy, heptyloxy et octyloxy,
et les halogènes présents dans la formule (I) peuvent être choisis parmi le brome, le chlore, le fluor et l'iode.

Par exemple, l'invention concerne l'utilisation pour l'obtention de compositions pharmaceutiques destinées à la prévention et au traitement des pathologies liées à l'échangeur Cl⁻/HCO₃⁻, Na⁺ indépendant, des composés de formule (I) correspondant aux formules (I/a) et (I/b), cas particuliers des composés de formule (I) : 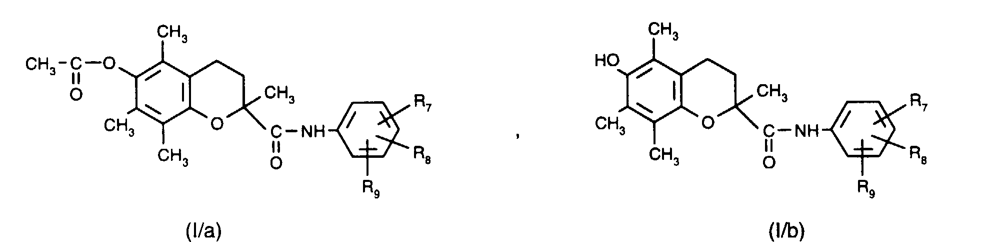
leurs énantiomères et diastéréoisomères,
et leurs sels d'addition à une base pharmaceutiquement acceptable.

Par exemple, l'invention concerne l'utilisation selon l'invention des composés choisis parmi
1 N-(2,4,6-triméthylphényl)-(6-acétoxy-3,4-dihydro-2,5,7,8-tétraméthyl-2H[1]-benzopyran-2-yl)carboxamide
   Point de fusion : 148-149°C
2) N-(2,4,6-triméthylphényl)-(6-hydroxy-3,4-dihydro-2,5,7,8-tétraméthyl-2H[1 ]-benzopyran-2-yl)carboxamide
   Point de fusion : 163-164°C
3) N-(3,4,5-triméthoxyphényl)-(6-acétoxy-3,4-dihydro-2,5,7,8-tétraméthyl-2H[1]-benzopyran -2-yl)carboxamide
   Point de fusion : 65-68°C
4) N-(3,4,5-triméthoxyphényl)-(6-hydroxy-3,4-dihydro-2,5,7,8-tétraméthyl-2H[1]-benzopyran -2-yl)carboxamide
   Point de fusion : 154-156°C
5) N-(4-hydroxy-2,3-diméthylphényl)-(6-acétoxy-3,4-dihydro-2,5,7,8-tétraméthyl-2H[1] benzopyran-2-yl)carboxamide
   Point de fusion : 230-232°C
6) N-(4-hydroxy-2,3-diméthylphényl)-(6-hydroxy-3,4-dihydro-2,5,7,8-tétraméthyl-2H[1]-benzopyran-2-yl)carboxamide
   Point de fusion : 182-183°C
7) N-(2-carboxy-4,5-diméthoxyphényl)-(6-acétoxy-3,4-dihydro-2,5,7,8-tétraméthyl-2H[1]benzopyran-2-yl)carboxamide
   Point de fusion : 254-256°C
8) N-(2-carboxy-4,5-diméthoxyphényl)-(6-hydroxy-3,4-dihydro-2,5,7,8-tétraméthyl-2H[1]benzopyran-2-yl)carboxamide
   Point de fusion : 244°C
9) N-(3,5-dichloro-4-hydroxyphényl)-(6-acétoxy-3,4-dihydro-2,5,7,8-tétraméthyl-2H[1]benzopyran-2-yl)carboxamide
   Point de fusion : 168°C
10) N-(3,5-dichloro-4-hydroxyphényl)-(6-hydroxy-3,4-dihydro-2,5,7,8-tétraméthyl-2H[1]benzopyran-2-yl)carboxamide
   Point de fusion : 208°C
11) N-(2-carboxy-4,6-diméthylphényl)-(6-acétoxy-3,4-dihydro-2,5,7,8-tétraméthyl-2H[1]benzopyran-2-yl)carboxamide
12) N-(2-carboxy-4,6-diméthylphényl)-(6-hydroxy-3,4-dihydro-2,5,7,8-tétraméthyl-2H[1]benzopyran-2-yl)carboxamide
   Point de fusion : 244°C
13) N-(2,4,5-tdméthylphényl)-(6-acétoxy-3,4-dihydro-2,5,7,8-tétraméthyl-2H[1]-benzopyran-2-yl)carboxamide
   Point de fusion : 160-162°C
14) N-(2,4,5-triméthylphényl)-(6-hydroxy-3,4-dihydro-2,5,7,8-tétraméthyl-2H[1]-benzopyran-2-yl)carboxamide
   Point de fusion : 180-182°C
15) N-(3,4,5-triméthoxyphényl)-(6-acétoxy-3,4-dihydro-5,7,8-triméthyl-2H[1]-benzopyran-2-yl)carboxamide
16) N-(3,4,5-triméthoxyphényl)-(6-hydroxy-3,4-dihydro-5,7,8-triméthyl-2H[1]-benzopyran-2-yl)carboxamide
   Point de fusion : 174-175°C
17) N-(3,4,5-triméthoxyphényl)-(6-acétoxy-7-tertio-butyl-3,4-dihydro-2-méthyl-2H[1]-benzopyran-2-yl)carboxamide
18) N-(3,4,5-triméthoxyphényl)-(6-hydroxy-7-tertio-butyl-3,4-dihydro-2-méthyl-2H[1]-benzopyran-2-yl)carboxamide
   Point de fusion : 212-213°C
19) N-(3,4,5-triméthoxyphényl)-(6-acétoxy-7-tertio-butyl-3,4-dihydro-2-méthyl-2H[1]-benzopyran-2-yl)acétamide
20) N-(3,4,5-triméthoxyphényl)-(6-hydroxy-7-tertio-butyl-3,4-dihydro-2-méthyl-2H[1]-benzopyran-2-yl)acétamide
   Point de fusion : 167-168°C
21) N-(3,5-diterfio-butyl-4-hydroxyphényl)-(6-acétoxy-3,4-dihydro-2,5,7,8-tétraméthyl-2H[1]-benzopyran-2-yl)carboxamide
22) N-(3,5-ditertio-butyl-4-hydroxyphényl)-(6-hydroxy-3,4-dihydro-2,5,7,8-tétraméthyl-2H[1]-benzopyran-2-yl)carboxamide
   Point de fusion : 172-174°C
23) N-(2,4,5-triméthylphényl)-(6-acétoxy-3,4-dihydro-5,7,8-triméthyl-2H[1]-benzopyran-2-yl)carboxamide
24) N-(2,4,5-triméthylphényl)-(6-hydroxy-3,4-dihydro-5,7,8-triméthyl-2H[1]-benzopyran-2-yl)carboxamide
25) N-(2,4,5-triméthylphényl)-(6-acétoxy-7-tertio-butyl-3,4-dihydro-2-méthyl-2H[1]-benzopyran-2-yl)carboxamide
26) N-(2,4,5-triméthylphényl)-(6-hydroxy-7-tertio-butyl-3,4-dihydro-2-méthyl-2H[1]-benzopyran-2-yl)carboxamide
27) N-(2,4,5-triméthylphényl)-(6-acétoxy-7-tertio-butyl-3,4-dihydro-2-méthyl-2H[1]-benzopyran-2-yl)acétamide
   et
28) N-(2,4,5-triméthylphényl)-(6-hydroxy-7-tertio-butyl-3,4-dihydro-2-méthyl-2H[1]-benzopyran-2-yl)acétamide ;
de leurs énantiomères ou diastéréoisomères,
et de leurs sels d'addition avec une base pharmaceutiquement acceptable.

Parmi les bases pharmaceutiquement acceptables que l'on peut utiliser pour former un sel d'addition avec les composés de l'invention, on peut citer à titre d'exemples et de façon non limitative, les hydroxydes de sodium, de potassium, de calcium, ou d'aluminium, les carbonates de métaux alcalins ou alcalinoterreux, et les bases organiques comme la triéthylamine, la benzylamine, la diéthanolamine, la tert-butylamine, la dicyclohexylamine, et l'arginine.

Les composés de formule (I) sont connus comme antioxydants et leur préparation est décrite dans EP 512899.

Les composés de formule (I) possèdent des propriétés pharmacologiques très intéressantes. Ils ont notamment montré une capacité inhibitrice très puissante de l'échangeur Cl⁻/HCO₃⁻, Na⁺ indépendant (ci-après dénommé AE (pour "Anion Exchanger"). Il est également possible d'utiliser, le cas échéant, une sélectivité des composés de formule (I) pour l'une ou l'autre des 3 isoformes de l'AE doublée ou non d'une sélectivité tissulaire pour une isoforme donnée. Cette sélectivité permet de garantir une spécificité dans l'action thérapeutique avec un meilleur ratio efficacité/sécurité.

L'évaluation pharmacologique des composés de formule (I) a notamment permis d'établir que les composés de formule (I) sont notamment utiles pour l'obtention de compositions pharmaceutiques destinées à la prévention et au traitement :
1- des pathologies du tractus gastro-intestinal, par exemple de l'ulcère gastroduodénal, des états d'hypersécrétion gastrique acide (oesophagite par reflux, syndrome de Zollinger-Ellison) et des troubles du transit.

En effet, la cellule pariètale gastrique possède un AE (type AE2) basolatéral dont l'activité est indispensable au maintien de la sécrétion acide gastrique par la H⁺/K⁺ ATPase apicale (Muallem S. et al. J. Biol. Chem. 1988, 263 : 14703-14711) et la cellule épithéliale du côlon possède un AE apical, impliqué dans la sécrétion d'eau et de bicarbonate, cet AE étant la cible de la toxine du choléra.
2- des pathologies de l'os, par exemple les troubles du remaniement osseux, notamment l'ostéoporose et la maladie de Paget, et les maladies inflammatoires avec destruction de l'os, notamment l'arthrite.

En effet l'ostéoclaste, cellule fondamentale impliquée dans la résorption osseuse possède un AE basolatéral dont l'activité est essentielle au maintien de la sécrétion acide ostéoclasique par la "vacuolar-type" H⁺-ATPase apicale située au niveau de la bordure en brosse, cette sécrétion acide étant indispensable à la destruction par l'ostéoclaste de la matrice osseuse (Teti A. et al. J. Clin. lnvest. 1989, 83 : 227-233 et Hall T.J. et al. Calcif. Tissue Int. 1989, 45 : 378-380).
3- des pathologies centrales et notamment des oedèmes cérébraux d'origine ischémique ou traumatique.

En effet, l'astrocyte possède un AE facilitateur de l'influx de Cl⁻ dans la cellule qui est impliqué dans la constitution de l'oedème astrocytaire (Mellegard P. et al. Acta Neurochir., 1994, 60 : 34-37). Un AE (type AE2) est également présent au niveau des plexus choroïdes et permet de moduler la sécrétion du liquide céphalorachidien (Lindsey A.E et al P.N.A.S. USA 1990, 87 : 5278-5282). L'existence d'un AE (AE3) au niveau neuronal permet une action sur le développement et la maturation neuronale (Raley-Susman K.M. et al Brain Res. 1993, 614 : 308-314).
4- des pathologies rénales et notamment des désordres de l'équilibre acido-basique (situations d'acidose et d'alcalose) et de l'équilibre hydroélectrolytique ainsi que des néphropathies, notamment diabétique.

En effet, l'AE joue un rôle important au niveau du tube collecteur, segment fondamental pour la régulation de l'équilibre acido-basique (Schuster V.L. Annu. Rev. Physiol. 1993, 55 : 267-288). Il existe notamment un AE basolatéral (AE1) au niveau des cellules intercalaires a ("acid secreting") impliqué dans l'acidification de l'urine et un AE apical au niveau des cellules intercalaires β ("base secreting") impliqué dans l'alcalinisation de l'urine (Weiner I.D. et al. Am. J. Physiol 1994, 267 : F952-F964). Il existe en outre au niveau de la cellule mésangiale un AE dont l'activité est régulée par les facteurs de croissance (Ganz M.B. et al. Nature 1989, 337 : 648-651).
5- des pathologies cardiovasculaires : ischémie, angor, arhythmies, insuffisance cardiaque, hypertension, états d'hyperprolifération / hypertrophie cardiovasculaire (athérosclérose, resténose).

En effet, l'action bénéfique des composés de formule (I) et notamment leur importance dans la protection et la restauration cardiovasculaire implique l'AE (AE3) du cardiomyocyte qui est activé en situation d'ischémie myocardique et contribue à l'acidification intracellulaire en complément de l'acidification métabolique déjà présente lors de l'ischémie (Yannoukakos D. et al. Circ Res, 1994, 75 : 603-614) différents médiateurs, accrus en situation d'ischémie (par exemple l'ATP et les catécholamines) pouvant en outre activer l'AE (Pucéat M. et al. J. Physiol. 1991, 444 : 241-256 et Désilets H. et al. Circ. Res. 1994, 75 : 862-869). Il est également connu que l'AE est régulé par les facteurs de croissance (Ganz M.B. et al. Nature, 1989, 337 : 648-651) et présente une hyperactivité au niveau des érythrocytes de l'hypertendu (Alonso A. et al. Hypertension 1993, 22 : 348-356).
6- de la mucoviscidose.
7- des pathologies pulmonaires, en raison de la présence d'un AE (AE2), régulateur du pH intracellulaire sur la surface basolatérale des cellules épithéliales alvéolaires (Lubman R.L. et al. Am. J. Respir. Cell. Mol. Biol. 1995, 12 : 211-219).
8- du diabète (insulinosécrétion) (Sheperd R.M. et al. J. Biol. Chem 1995, 270 : 7915-7921 et Sheperd R.M. et al. Diabetologia 1995, 38 : A119, abrégé 461) et des complications diabétiques, notamment, les pathologies oculaires du diabétique telles que la rétinopathie et les neuropathies sensitives et motrices telle que la gastroparésie diabétique.
9- de la cytotoxicité induite par les anticancéreux, telle que l'adriamycine (Asaumi J-I et al. Anticancer Research, 1995, 15 : 71-76).
10- et de la maladie d'Alzeimer (Renkawek K et al. Neuroreport, 1995, 6 : 929-932).

L'invention concerne ainsi l'utilisation des composés de formule (I) pour l'obtention de compositions pharmaceutiques destinées à la prévention et au traitement des pathologies du tractus gastro-intestinal, des pathologies de l'os, des pathologies centrales et notamment des oedèmes cérébraux d'origine ischémique ou traumatique, des pathologies rénales et notamment des désordres de l'équilibre acido-basique (situations d'acidose et d'alcalose) et de l'équilibre hydroélectrolytique ainsi que des néphropathies, des pathologies cardiovasculaires, de la mucoviscidose, des pathologies pulmonaires, du diabète et des complications diabétiques, de la cytotoxicité induite par les anticancéreux et de la maladie d'Alzheimer.

Les composés de formule (I) ont également montré une utilité pour l'obtention de compositions pharmaceutiques destinées aux conditions d'hyperprolifération, par exemple dans le cadre des cicatrisations et des réparations tissulaires, l'AE étant impliqué dans la régulation de la croissance cellulaire (Ganz et al. Nature 1989, 337 : 648-651).

L'invention s'étend également à l'utilisation des composés de formule (I) comme outils pharmacologiques dans le domaine pharmacologique de l'échangeur Cl⁻/HCO₃⁻, Na⁺ indépendant.

Notamment, l'invention s'étend à l'utilisation des composés de formule (I) comme outils pharmacologiques :
- pour détecter l'expression, par exemple tissulaire, de l'AE,
- pour réaliser des études de liaisons à l'AE,
- pour identifier et localiser de nouvelles isoformes de l'AE,
- pour cloner, séquencer et exprimer l'AE.

La présente invention a également pour objet les compositions pharmaceutiques contenant un des dérivés de formule (I), ou un de ses sels d'addition à une base pharmaceutiquement acceptable en combinaison avec un ou plusieurs excipients ou vecteurs pharmacologiquement acceptables.

Parmi les compositions pharmaceutiques selon l'invention, on pourra citer, à titre d'exemples et de façon limitative, celles qui conviennent à l'administration orale, parentérale, nasale, rectale, perlinguale, oculaire ou pulmonaire et notamment les préparations injectables, les aérosols, les gouttes oculaires ou nasales, les comprimés simples, pelliculés ou dragéifiés, les gélules, les capsules, les suppositoires, les crèmes, pommades, et les gels dermiques.

Les ingrédients présents dans la composition pharmaceutique sont choisis par exemple parmi
a) des diluants, comme le lactose, le dextrose, le sucrose, le mannitol, le sorbitol, la cellulose et la glycérine ;
b) des lubrifiants, comme la silice, le talc, l'acide stéarique et ses sels de magnésium et de calcium et le polyéthylène glycol ;
c) des liants, comme le silicate d'aluminium et de magnésium, l'amidon, la gélatine, le tragacanthe, la méthylcellulose, la carboxyméthylcellulose de sodium et la polyvinylpyrrolidone ;
d) des désintégrants, comme l'agar, l'acide alginique et son sel de sodium et les mélanges effervescents ;
   et
e) les absorbants, les colorants, les aromates et les édulcorants.

Les solutions injectables sont de préférence des solutions isotoniques aqueuses ou des suspensions et les suppositoires sont avantageusement préparés à partir d'émulsions ou de suspensions grasses.

Les compositions peuvent être stérilisées et/ou contenir des adjuvants tels que des conservateurs, des agents mouillants, solubilisants ou émulsifiants, des sels pour réguler la pression osmotique ou des tampons.

La posologie utile varie selon le sexe, l'âge et le poids du patient, la voie d'administration, la nature de l'affection et des traitements éventuellement associés et s'échelonne entre 0,1 mg et 1 g par 24 heures, par exemple entre 1 et 100 mg par 24 heures, plus particulièrement entre 10 et 50 mg par 24 heures, par exemple 20 mg par 24 heures.

Les exemples qui suivent illustrent l'invention et ne la limitent en aucune façon.

### Exemple A : MESURE DE L'ACTIVITE DE L'ECHANGEUR Cl⁻/HCO₃⁻, Na⁺ INDEPENDANT DANS LES CARDIOMYOCYTES VENTRICULAIRES ISOLES DE COEUR DE RAT

L'étude est réalisée sur des cardiomyocytes ventriculaires isolés de coeur de rat par dissociation enzymatique. Le pH intracellulaire d'une cellule unique est mesuré par le marqueur fluorescent carboxy-SNARF-1 dont la fluorescence dépend de son état de protonation (Buckler K.J. et al. Pflügers Archiv, 1990, 417 : 234-239). Les cellules sont préalablement "chargées" en SNARF-1-AM (forme estérifiée et perméante du marqueur) et la fluorescence du SNARF intracellulaire est suivie à l'aide d'un microscope inversé, équipé en épifluorescence. Les mesures sont converties en pH en utilisant un fichier de calibration.

L'activité de l'échangeur Cl⁻/HCO₃⁻, Na⁺ indépendant est étudiée après activation par induction d'une charge alcaline des cellules par la méthode à l'acétate (Roos A et al. Physiol. Rev., 1981, 61 : 296-434).

Les activités de l'échange Na⁺/H⁺ et du cotransport Na⁺/HCO₃⁻ sont étudiées après activation par induction d'une charge acide des cellules par la méthode à l'ammonium (Boron W.F. et al. J. Gen. Physiol., 1976, 67 : 91-112).

Les composés selon l'invention sont utilisés à des concentrations de 10⁻¹¹ à 10⁻⁵M et leur IC₅₀ (concentration inhibitrice 50 %) est calculée pour chaque transporteur ionique. En parallèle les effets des molécules sur le pouvoir tampon intrinsèque intracellulaire et le pH intracellulaire de repos sont déterminés.

Les composés de formule (I) se sont révélés posséder une puissante capacité inhibitrice de l'échangeur Cl⁻/HCO₃⁻, Na⁺ indépendant. Par exemple, le composé de l'exemple 14 est un inhibiteur sélectif de l'échangeur Cl⁻/HCO₃⁻, Na⁺ indépendant avec une IC₅₀ de l'ordre de 10-¹⁰M.

### Exemple B : ULCERE GASTROINTESTINAL

- Sur culture de cellules pariétales de lapin : on mesure l'activité de l'AE et la sécrétion acide dans des conditions de base ou de stimulation par des sécrétagogues (Muallem S. et al., J. Biol. Chem., 1988, 263 : 14703-14711).
- Ulcère de stress chez le rat : le modèle combine une immobilisation (boîte à contention) et une immersion partielle du rat dans de l'eau à 22°C pendant 6 heures. La sévérité des lésions gastriques est appréciée chez des rats contrôles ou traités préventivement par les composés utilisés selon l'invention 30 minutes avant l'exposition au stress (Takagi K. et al., Japan J. Pharmacol. 1968, 18 : 9-18).

### Exemple C : TROUBLES DU REMANIEMENT OSSEUX

- Sur culture d'ostéoclastes de poulet : on mesure l'activité de l'AE et la sécrétion acide (Teti A et al, J. Clin. Invest., 1989, 83 : 227-233).
- Test de résorption osseuse in vitro : des ostéoclastes de lapins nouveaux nés sont placés en contact avec des tranches osseuses de boeuf et incubés pendant 6 heures avec les composés utilisés selon l'invention. Après incubation chaque tranche osseuse est examinée pour évaluer l'intensité de la résorption osseuse (Chambers T.J. et al. Endocrinology, 1985, 116 : 234-239).

### Exemple D : OEDEME CEREBRAL

- Sur culture d'astrocytes de rat : on mesure l'activité de l'AE (Mellegard P. et al. Acta Neurochir., 1994, 60 : 34-37).
- Sur culture d'astrocytes de rat : on mesure le gonflement astrocytaire induit par l'hypoxie in vitro : l'étude est réalisée sur des astrocytes en culture primaire obtenus à partir de cortex cérébraux de rats nouveaux-nés Sprague-Dawley et soumis à une hypoxie pendant 18 heures. L'effet anti-oedémateux est étudié principalement en mesurant le volume cellulaire en présence de 3-O-méthyl-D-glucose radioactif et l'activité extracellulaire en lactate déshydrogénase, marqueur de l'altération membranaire (Kimelberg H.K. et al. Mol. Chem. Neuropath., 1989, 11 : 1-31).

### Exemple E : ISCHEMIE MYOCARDIQUE

Modèle d'ischémie-reperfusion sur coeur isolé de rat : le coeur isolé de rat est perfusé en mode Langendorff. Une ischémie locale d'une durée de 10 minutes est obtenue par ligature de l'artère coronaire gauche, suivie d'une période de reperfusion par levée de la ligature. Les paramètres fonctionnels (débit coronaire, pression intraventriculaire gauche et fréquence cardiaque) sont mesurés toutes les deux minutes et les troubles du rythme sont évalués à la reperfusion. Les composés testés sont perfusés pendant toute la durée de la séquence d'ischémie-reperfusion. (Rochette L. et al, Clin. Exp. Theory. Practice, 1987, A9 365-368).

### Exemple F : ETUDE CARDIOVASCULAIRE

Les composés sont testés à une concentration de 0,1 µM dans une solution de DMSO dans du tampon de Krebs (0,01 % v/v) sur les arhythmies. 6 coeurs de rats isolés ont été utilisés pour le composé à tester et 6 pour le contrôle. On réalise une perfusion en mode Langendorff (Rees S.A. et Curtis M.J. J. Card. Pharmacol. 1995, 26 : 280-288).

Les résultats préliminaires (n = 6) montrent que le composé de l'exemple 14 permet de réduire de 50 % l'incidence de la fibrillation ventriculaire observée pendant l'ischémie, comparativement aux coeurs contrôles. Il possède donc une activité cardioprotectrice. De même, ce composé réduit de 50 % l'incidence de la fibrillation ventriculaire observée lors de la reperfusion.

Il s'avère donc que le composé de l'invention possède une bonne activité sur le système cardiovasculaire. Il possède notamment une puissante activité antifibrillante.

### Exemple G : COMPOSITION PHARMACEUTIQUE : COMPRIMES

Comprimés dosés à 20 mg de N-(2,4,5-triméthylphényl)-(6-hydroxy-3,4-dihydro-2,5,7,8-tétraméthyl-2H[1]-benzopyran-2-yl)carboxamide.

### Formule de préparation pour 1000 comprimés

| | |
|---|---|
| N-(2,4,5-triméthylphényl)-(6-hydroxy-3,4-dihydro-2,5,7,8-tétraméthyl-2H[1]-benzopyran-2-yl)carboxamide | 20 g |
| Amidon de blé | 240 g |
| Lactose | 180 g |
| Stéarate de magnésium | 2,0 g |
| Silice | 0,8 g |
| Hydroxypropylcellulose | 2,0 g |

## Revendications

1. Utilisation des composés de formule (I) : 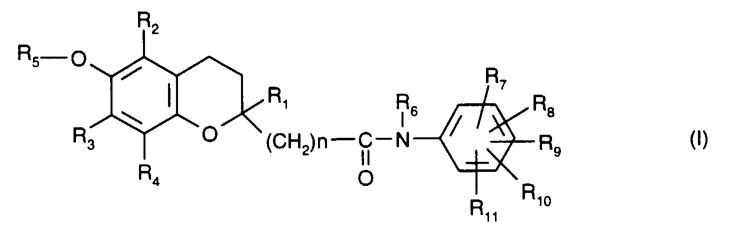
dans laquelle
n représente 0 ou 1,
R₁, R₂, R₃ et R₄, identiques ou différents, représentent, chacun indépendamment l'un de l'autre, un hydrogène ou un alkyl ;
R₅ représente un hydrogène ou un radical choisi parmi alkyl, alkylcarbonyl, alkoxyalkyl, alkoxycarbonyl, alkoxycarbonylalkyl et carboxyalkyl ;
R₆ représente un hydrogène ou un radical choisi parmi alkyl, phényl et phénylalkyl ;
R₇, R₈ et R₉ représentent, chacun indépendamment l'un de l'autre, un radical choisi parmi halogène, alkyl, alkyl substitué par un ou plusieurs halogènes, alkoxy, hydroxy, alkoxycarbonyl et carboxy ;
et R₁₀ et R₁₁ représentent, chacun indépendamment l'un de l'autre, un hydrogène ou un radical choisi parmi halogène, alkyl, alkyl substitué par un ou plusieurs halogènes, alkoxy, hydroxy, alkoxycarbonyl et carboxy ;
leurs énantiomères et diastéréoisomères et leurs sels d'addition à une base pharmaceutiquement acceptable,
étant entendu que
les termes "alkyl" et "alkoxy" désignent des groupements linéaires ou ramifiés de 1 à 8 atomes de carbone,
pour l'obtention de compositions pharmaceutiques destinées à la prévention et au traitement des troubles liés à l'échangeur Cl⁻/HCO₃⁻, Na⁺ indépendant.

2. Utilisation selon la revendication 1 des composés de formule (I) selon la revendication 1 correspondant aux formules (I/a) et (I/b), cas particuliers des composés de formule (I) : 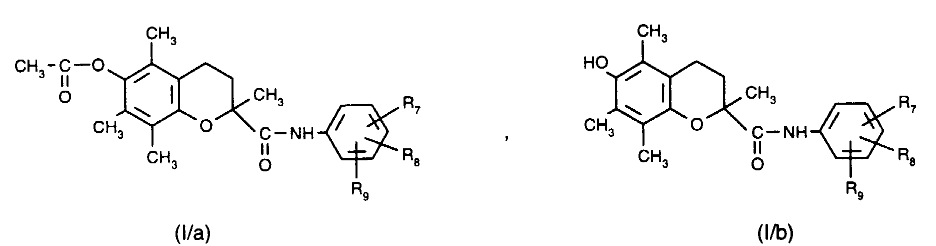 leurs énantiomères et diastéréoisomères,
et leurs sels d'addition à une base pharmaceutiquement acceptable.

3. Utilisation selon la revendication 1 du N-(2,4,6-triméthylphényl)-(6-acétoxy-3,4-dihydro-2,5,7,8-tétraméthyl-2H[1 ]-benzopyran-2 yl)carboxamide.

4. Utilisation selon la revendication 1 du N-(3,5-dichloro-4-hydroxyphényl)-(6-hydroxy-3,4-dihydro-2,5,7,8-tétraméthyl-2H[1]-benzopyran-2-yl)carboxamide.

5. Utilisation selon la revendication 1 du N-(2,4,5-triméthylphényl)-(6-hydroxy-3,4-dihydro-2,5,7,8-tétraméthyl-2H[1]-benzopyran-2-yl)carboxamide.

6. Utilisation des composés de formule (I) selon la revendication 1 pour l'obtention de compositions pharmaceutiques destinées à la prévention et au traitement des pathologies du tractus gastro-intestinal, des pathologies de l'os, des pathologies centrales et notamment des oedèmes cérébraux d'origine ischémique ou traumatique des pathologies rénales et notamment des désordres de l'équilibre acido-basique (situations d'acidose et d'alcalose) et de l'équilibre hydroélectrolytique ainsi que des néphropathies, des pathologies cardiovasculaires, de la mucoviscidose et des pathologies pulmonaires, du diabète et des complications diabétiques, de la cytotoxicité induite par les anticancéreux et de la maladie d'Alzheimer.

7. Utilisation selon la revendication 6 pour l'obtention de compostions pharmaceutiques destinées à la prévention et au traitement des pathologies cardiovasculaires : ischémie, angor, arhythmies, insuffisance cardiaque, hypertension, états d'hyperprolifération / hypertrophie cardiovasculaire (athérosclérose, resténose).

8. Utilisation selon la revendication 1 des composés de formule (I) pour l'obtention de compositions pharmaceutiques destinées aux conditions d'hyperprolifération.

9. Utilisation des composés de formule (I) selon la revendication 1 comme outils pharmacologiques dans le domaine pharmacologique de l'échangeur Cl⁻/HCO₃⁻, Na⁺ indépendant.

10. Utilisation selon la revendication 9 des composés de formule (I) selon la revendication 1 comme outils pharmacologiques pour détecter l'expression, par exemple tissulaire, de l'échangeur Cl⁻/HCO₃⁻, Na⁺ indépendant ; pour réaliser des études de liaisons à l'échangeur Cl⁻/HCO₃⁻, Na⁺ indépendant ; pour identifier et localiser de nouvelles isoformes de l'échangeur Cl⁻/HCO₃⁻, Na⁺ indépendant ; pour cloner, séquencer et exprimer l'échangeur Cl⁻/HCO₃⁻, Na⁺ indépendant.

11. Compositions pharmaceutiques contenant un des dérivés de formule (I), utilisés selon la revendication 1 ou un de ses sels d'addition à une base pharmaceutiquement acceptable en combinaison avec un ou plusieurs excipients ou vecteurs pharmacologiquement acceptables.
